Europäisches Patentamt

(19) European Patent Office

Office européen des brevets

(11) Publication number : 0 390 849 B1

# EUROPEAN PATENT SPECIFICATION

(12)

(45) Date of publication of patent specification :
07.01.93 Bulletin 93/01

(51) Int. Cl.⁵ : **A61K 37/02, A61K 37/66, A61K 31/70**

(21) Application number : 89900745.4

(22) Date of filing : 30.11.88

(86) International application number :
PCT/US88/04267

(87) International publication number :
WO 89/05149 15.06.89 Gazette 89/13

(54) **METHYL CELLULOSE PHARMACEUTICAL COMPOSITION.**

(30) Priority : 03.12.87 US 128391

(43) Date of publication of application :
10.10.90 Bulletin 90/41

(45) Publication of the grant of the patent :
07.01.93 Bulletin 93/01

(84) Designated Contracting States :
AT BE CH DE FR GB IT LI LU NL SE

(56) References cited :
EP-A- 0 177 223
EP-A- 0 225 130
WO-A-83/01198
US-A- 4 359 483
US-A- 4 609 546
US-A- 4 680 175
US-A- 4 708 861
US-A- 4 717 717
US-A- 4 762 720
US-A- 4 762 915

(56) References cited :
Science, Vol. 236, Issued June 1987; "The
Human Hematopoietic Colony-Stimulating
Factors," (CLARK), pages 1229-37
Blood, vol. 67, issued February 1986, "The
Molecular Biology and Functions of the
Granulocyte-Macrophage Colony -Stimulati-
ngFactors," (METCALF), pages 257-67
Seminars in Oncology; Vol. 13, issued June
1986, "Future Perspectives for Biological Res-
ponse Modifiers,"(MIHICH), pages234-54

(73) Proprietor : THE LIPOSOME COMPANY, INC.
One Research Way Princeton Forrestal Center
Princeton, NJ 08540 (US)

(72) Inventor : ESTIS, Leonard, F.
56 Grafton Road
Upton, MA 01568 (US)
Inventor : KEYES, Lynn, D.
56 Grafton Road
Upton, MA 01568 (US)
Inventor : RECINE, Marie, S.
19 Hoffman Drive
Hamilton Twp., NJ 08690 (US)

(74) Representative : Warcoin, Jacques
Cabinet Régimbeau 26, avenue Kléber
F-75116 Paris (FR)

## Description

This invention concerns a pharmaceutical composition comprising methyl cellulose and an immunomodulator (such as interleukin) providing prolonged elaboration of the immunomodulator in a subject animal, and method of preparation.

A number of peptide immunomodulators ("immunomodulators") such as colony stimulating factor (e.g., granulocytic CSF and monocytic CSF), interferons (currently including alpha through gamma) and interleukins have recently been identified as clinically useful and are now available in commercial quantities.

The family of interleukins, currently comprising the IL1, IL2, IL3 and IL4 peptides, has recently become the object of therapeutic interest as a result of advances in the techniques of isolation and production of peptides.

Interleukin, particularly IL2, is thought to be of therapeutic effect in the treatment of certain pathologies such as neoplasms and immunodeficiencies (e.g. AIDS) in animals, including humans. Among the problems associated with the therapeutic use of interleukin (and other peptide immunomodulators) are maintenance of therapeutically effective blood levels of immunomodulators in the subject animal being treated, toxicity of the immunomodulator, and the interrelated problem of short half-life of immunomodulator (possibly as a result of the action of proteolytic enzymes) in requiring large and relatively toxic amounts of immunomodulator per dose to yield suitable duration of therapeutically effective plasma levels. Interleukin is a labile peptide subject to breakdown in and excretion from the in vivo physiological environment. Some investigators have reported that 95'% of free interleukin administered to an animal is lost within 1 hour after administration. Donohue, J.H. and Rosenberg, S.A., "The Fate of Interleukin-2 after in vivo Administration", Journal of Immunology, 130:2203 (1983); Chang, A.E., Hyatt, C.L., and Rosenberg, S.A., "Systemic Administration of Recombinant Human Interleukin-2 in Mice", Journal of Biological Response Modifiers, 3:561 (1984).

The general use of gels including methyl cellulose in the administration of therapeutic agents has been reported in U.S. Patent Ser. No. 4,708,861 issued November 24, 1987 to Popescu et al.

It has now been discovered that immunomodulators like interleukin dispersed specifically in methyl cellulose suprisingly remains present and biologically active for up to 12 days or longer subsequent to administration. This effect is not found with other cellulosic materials.

This invention will be understood to include a pharmaceutical composition of prolonged elaboration comprising methyl cellulose and an immunomodulator. The immunomodulator is encapsulated in a liposome, such as an SPLV, a FATMLV, an SUV, a LUVET, an MPV, or an MLV.

In particular embodiments the immunomodulator is a colony stimulating factor, an interferon, or an interleukin, particularly an interleukin such as IL2. In some applications IL2 is present in from 0.5µg/mg to 600µg/mg methyl cellulose.

Further included is methyl cellulose from 1Pa x sec 1,000 to 10Pa x sec (10,000cp), particularly from 1Pa x sec (1,000 cp to 4Pa x sec (4,000cp), and further wherein the methyl cellulose is a gel. Preferred gel is from 1% to 15% (w/v) methyl cellulose, and more preferred from 3% to 12% w/v (particularly 6% w/v), and yet further preferred wherein the methyl cellulose is 1,500cp and also 6% w/v. Methyl cellulose of 1.500cp and 6% w/v is preferred for IL2.

This invention includes a method of preparing a pharmaceutical composition of prolonged elaboration comprising admixing methyl cellulose and an immunomodulator, and further includes forming a gel of methyl cellulose. In one embodiment the admixing is performed at a temperature at least below 10°C. In this method the immunomodulator is encapsulated in a liposome, such as by forming an SPLV, a FATMLV, an SUV, a LUVET, an MPV, or an MLV.

Included in the method is methyl cellulose from 1Pa x sec (1,000) to 10Pa x sec (10,000cp), and particularly from 1Pa x sec (1,000) to 4Pa x sec 4,000cp, and most particularly 1,5Pa x sec (1,500cp).

Also included in the is wherein the methyl cellulose being a gel.

This includes the methyl cellulose being from 1% to 15% w/v (particularly at 1,5Pa x sec (1,500cp), and wherein the methyl cellulose being from 3% to 12% w/v and particularly wherein the gel is 6% w/v.

Further included in this invention is the method of prolonged administration of an immunomodulator to an animal comprising administering said immunomodulator in methyl cellulose includes wherein the immunomodulator is a colony stimulating factor, an interferon, or an interleukin, and particularly interleukin such as IL2. Also included is wherein the immunomodulator is encapsulated in a liposome. Included as to IL2 in the practice of this method of is IL2 administered from 0.25µg to 2µg/kg animal weight or wherein IL2 is present in from 0.5µg to 600µg/mg methyl cellulose. In a particular embodiment of this method is the methyl cellulose. In a particular embodiment of this method is the methyl cellulose being 1,5Pa x sec (1,500cp) and 6% (w/v).

In WO 83/01198, a composition of a gel of methyl cellulose and interferon is described.

Immunomodulator (in some citations "immunomoderator") will be understood to mean peptides or proteins which posotively or negatively regulates the immune response of an animal to foreign antigens, such as cells, bacteria, viri and parasites. Exemplary of such immunomodulators are colony stimulating factor, interferon, and interleukin as well as analogues and derivatives thereof.

Interleukin shall be understood to include all interleukins such as IL1, IL1a, IL2, IL3, IL4, and analogues and derivatives thereof.

Interleukin is available from several sources such as ICN Biochemical, Cleveland, Ohio (IL1, IL2, IL3) and Amgen Biologicals, Thousand Oaks, Califomia (IL2 ).

Methyl cellulose shall be understood to mean a methyl ether of cellulose (long chain substituted cellulose ether of 50-1500 anhydrous units containing 26-32% methoxyl groups).

Methyl cellulose is available from many sources and in many forms and at viscosities of from 0,01 to 10 Pa.s (10 to 10,000cp) (e.g., powdered methyl cellulose is available from Sigma Chemical Co., St. Louis, MO.). Preferred in this invention is are the viscosities in the 1,5 to 4 Pa.s (1,500 to 4,000cp) range, with 1,5 Pa.s (1,500cp) most preferred.

Methyl cellulose in powdered form is suitable to form a gel composition of this invention. This is conveniently accomplished in boiling water or buffer however lower temperatures may be used with vigorous mixing. Gel concentrations of 1% to 15% (w/v) are useful. With 1,5 Pa.s (1,500cp) methyl cellulose from 3% to 12% concentrations are preferred. To avoid methyl cellulose accumulation at the site of injection, the lowest concentration offering adequate immunomodulator retention is preferred.

Mixing of the immunomodulator and the methyl cellulose in the form of a gel is suitable to form the composition of this invention. This is conveniently accomplished at a reduced temperature, below 10°c and above the freezing point if the gel (at least as low as 0°C), and preferably 4°C. Lower nonfreezing temperatures are preferred to minimize loss of immunomodulator activity. Care must be taken so that the immunomodulator is not denatured or deactivated by the process.

When a powdered methyl cellulose was used, the powder was first hydrated by adding it to boiling water, stirring to moisten and immediately cooling the reaction vessel in a heat sink such as ice. After cooling the immunomodulators such as interleukin, conviently in solution, may be combined with the methyl cellulose, conveniently with thorough mixing, such as by paddle mixer or vortexing.

In practice it was convenient to add higher concentration interleukin solution than that anticipated for the final composition to allow for dilution of the final product with the methyl cellulose.

In one embodiment the immunomodulator may be liposomally encapsulated prior to admixture with methyl cellulose. Long term elaboration of immunomodulator is provided by this embodiment, but the initial plasma level profile is somewhat reduced, as compared to free immunomodulator in methyl cellulose.

Liposomes are completely closed lipid bilayer membranes containing an entrapped aqueous volume. Liposomes may be unilamellar vesicles (possessing a single bilayer membrane ) or multilameller vesicles (onion-like structures characterized by multiple membrane bilayers, each separated from the next by an aqueous layer). The bilayer is composed of two lipid monolayers having a hydrophobic "tail" region and a hydrophilic "head" region. The structure of the membrane bilayer is such that the hydrophobic (nonpolar) "tails" of the lipid monolayers orient toward the center of the bilayer while the hydrophilic "head" orient towards the aqueous phase.

The original liposome preparation of Bangham, et al. (J. Mol, Biol., 1965, 12:238-252) involves suspending phospholipids in an organic solvent which is then evaporated to dryness leaving a phospholipid film on the reaction vessel. Next, and appropriate amount of aqueous phase is added, the mixture is allowed to "swell," and the resulting liposomes which consist of multilamellar vesicles (MLVs) are dispersed by mechanical means. This technique provides the basis for the development of the small sonicated unilamellar vesicles (SUVs) described by Papahadjopoulos et al. (Biochim. Biophys. Acta., 1968, 135:624-638), and large unilamellar vesicles.

Unilamellar vesicles may be produced using an extrusion apparatus by a method described in Cullis et al., PCT Application No. WO 87/00238, published January 16, 1986, entitled "Extrusion Technique for Producing Unilamellar Vesicles" incorporated herein by reference. Vesicles made by this technique, called LUVETS, are extruded under pressure through a membrane filter.

Monophasic vesicles (MPVs) are a particularly stable type of liposome described in U.S. Patent Ser. No. 4,588,578 to Fountain et al.

Another class of liposomes are those characterized as having substantially equal lamellar solute distribution. This class of liposomes is denominated as stable plurilamellar vesicles (SPLV) as defined in U.S. Patent No. 4,522,803 to Lenk, et al., monophasic vesicles as described in U.S. Patent No. 4558,579 to Fountain, et al. and frozen and thawed multilamellar vesicles (FATMLV) wherein the vesicles are exposed to at least one freeze and thaw-cycle; this procedure is described in Bally et al., PCT Publication No. 87/00043, January 15, 1987, entitled "Multilamellar Liposomes Having Improved Trapping Efficiencies".

A variety of sterols and their water soluble derivatives have been used to form liposomes; see specifically Janoff et al., PCT Publication No. WO 85/04578, published October 24, 1985, entitled "Steroidal Liposomes."

Mayhew et al., PCT Publication No. WO 85/00968, published March 14, 1985, described a method for reducing the toxicity of drugs by encapsulating them in liposomes comprising alpha-tocopherol and certain derivatives thereof. Also, a variety of tocopherols and their water soluble derivatives have been used to form liposomes, see Janoff et al., PCT Publication No. WO 87/02219, published April 23, 1987, entitled "Alpha Tocopherol-Based Vesicles."

The best results of admixing interleukin with methyl cellulose were obtained with 6% w/v and 1,5 Pa.s (1,500cp) methyl cellulose exhibiting retention/elaboration through over 10 days after intramuscular injection.

Liposomal encapsulation did not reduce the retention and release of the immunomodulator interleukin in various gels The most prolonged elaboration of interleukin was provided by methyl cellulose, particularly 1,5 Pa.s (1,500cp), and most particularly of 6% to 12%. These formulations substantially exceded the elaboration of interleukin from ultra-high viscosity carboxyvinyl polymers (HIVIS-A is the polymer suspended in 5mM ammonium acetate, approximately 200 Pa.s (200,000cp); HIVIS-G is the polymer suspended in glycerol approximately 1500 Pa.s (1,500,000cp) other, or any, known method.

The term prolonged elaboration as used herein is understood to mean the release of therapeutic agents from liposomal encapsulation over a period in excess of 24 hours, preferably at least 3 days, and in some embodiments as long as 2 weeks.

Methly cellulose substantially extended the elaboration of interleukin, and did so at elevated plasma levels, beyond the 3 day level seen for free interleukin or 7 day elaboration for liposomal interleukin without methyl cellulose.

The composition of the present invention can be used therapeutically in animals such as mammals, including man, in the treatment of infections or conditions which require the sustained delivery of immunomodulators in bioactive form. Such conditions include but are not limited to disease states such as cancer, AIDS and other immunodeficiencies. Each immunomodulator will exhibit prolonged therapeutic activity consonant with the particular immunomodulator.

The mode of administration of the preparation may determine the sites and cells in the organism to which the compound will be delivered. The composition of the present invention can be administered alone but may be administered in admixture with a pharmaceutical carrier selected with regard to the intended route of administration and standard pharmaceutical practice. The preparations may be injected parenterally, for example, or intra-arterially or intravenously (if not too viscous). The preparations may also be administered via intraperitoneal, subcutaneous, or intramuscular routes. For parenteral administration, they can be used, for example, in the form of a sterile aqueous solution which may contain other solutes, for example, enough salts or glucose to make the solution isotonic. Other uses, depending upon the particular properties of the preparation, may be envisioned by those skilled in the art.

For administration to humans in the curative treatment of disease states responding to immunomodulator therapy, the prescribing physician will ultimately determine the appropriate dosage for a given human subject, and this can be expected to vary according to the age, weight, and response of the individual as well as the nature and severity of the subject's disease. The dosage of the immunomodulator in liposomal form will generally be about that employed for the free immunomodulator. In some cases, however, it may be necessary to administer dosages outside these limits.

Dosage and administration of immumomodulator, in this invention, presumes therapeutically effective amounts. Therapeutically effective amounts of immunomodulator as used herein will mean that amount of immunomodulator that produces therapeutic action. This amount will be understood to vary with the particular immunomodulator or analog or derivative thereof, the condition being treated, the site, manner and duration of administration and other considerations known to those skilled in the art.

The compositions of this invention possess valuable pharmacological properties. They afford prolonged elaboration of immunomodulators including interleukins in a subject animal and provide antiviral, anticancer and antiAIDs efficacy in human and veterinary medicine. Effectiveness as to IL2 can be demonstrated, for example, using the the composition of this invention in a chemotherapy regimen, such as by weekly intramuscular injection at a concentration of 5µg to 60µg of interleukin/mg methyl cellulose in a dosage of from 0.25 to 2µg/kg subject weight.

Thus, depending on the immunomodulator, these compositions can be used in both antiviral and anticancer applications.

For interleukin the compositions are particularly useful as antitumor agents.

The compositions of this invention are generally administered to mammals, including but not limited to humans.

The pharmacologically active compositions of this invention can be processed in accordance with conventional methods of galenic pharmacy to produce medicinal agents for administration to subjects, e.g., mammals including humans in need of such treatment.

The compositions of this invention can be employed in admixture with conventional excipients, i.e., pharmaceutically acceptable organic or inorganic carrier substances suitable for parenteral, enteral (e.g., oral or inhalation) or topical application which do not deleteriously react with the active compositions. Suitable pharmaceutically acceptable carriers include but are not limited to water and salt solutions. The pharmaceutical preparations can be sterilized and if desired mixed with auxiliary agents, e.g., preservatives, stabilizers, wetting agents, emulsifiers, salts for influencing osmotic pressure or buffers which do not deleteriously react with the active compounds. They can also be combined where desired with other active agents, e.g., chemotherapeutics.

Generally, the compositions of this invention are dispensed in unit dosage form. For IL2 unit dosage form comprises 5 to 60μg interleukin in methyl cellulose carrier per unit dosage.

As to interleukin, the dosage of the compositions according to this invention generally are 0.25 to 2μg/kg/wk, preferably 0.5 to 1μg/kg/wk, when administered to subjects, e.g., humans to treat (e.g., cancer or viral infection) analogously to the known agent IL2.

It will be appreciated that the actual preferred amounts of active compositions in a specific case will vary according to the specific compositions being utilized, the particular compositions formulated, the mode of application, and the particular situs and organism being treated. Dosages for a given host can be determined using conventional considerations, e.g., by customary comparison of the differential activities of the subject compositions and of a known agent, e.g., by means of an appropriate, conventional pharmacological protocol.

## Example 1

### Free IL2 in Methyl Cellulose

Powdered methyl cellulose (1,5 Pa.s)(1500cp), 60mg, was weighed into a 1.4ml eppendorf tube. To this was added 0.6ml of boiling 5mM ammonium acetate, pH 5.0, and the resulting mixture stirred. After stirring the mixture was placed on ice and cooled to 4°C or until the mixture appeared transparent.

To the cooled mixture 133ul of IL2 at 3mg/ml was added and the mixture stirred. The volume was then brought up to 1.0ml with buffer.

For administration, the mixture, a methyl cellulose gel containing IL2 was loaded into a 1ml syringe which was centrifuged to remove bubbles. The loaded syringe was placed at 45°C for 1 hour prior to injection.

## Example 2

### Liposomal IL2 in Methyl Cellulose

Methyl cellulose was prepared according to the procedure of Example 1. Powdered methyl cellulose (1,5 Pa.s)(1500cp), 60mg, was weighed into a 1.4ml eppendorf tube. To this was added 0.6ml of boiling 5mM ammonium acetate, pH 5.0, and the resulting mixture stirred. After stirring the mixture was placed on ice and cooled to 4°C or until the mixture appeared transparent.

To the cooled mixture of methyl cellulose and water, 0.4mg IL2 encapsulated in LUVETS (by the method described in Cullis et al., PCT Application No. WO 87/00238, published January 16, 1986, entitled "Extrusion Technique for Producing Unilamellar Vesicles") at a concentration of 25μg IL2/mg of lipid (egg phosphatidylcholine) was added and the mixture stirred. Specifically, the LUVETS were prepared from SPLVs by first drying down the lipid (egg phosphatidylcholine) in a round bottom flask, resuspending in ether and IL2 at a ratio of 12:1 lipid to IL2 (200mg EPC: 17mg IL2), and sonicating the mixture while evaporating off the ether with nitrogen gas. The SPLVs, resuspended in a minimal volume of buffer to form a paste, were then passed through successively smaller filters in a LUVET apparatus (Lipex Biomembrane, Vancouver, B.C.) starting at 0.4μm and ending with 10 passes through a 0.1μm filter. The resulting LUVETs where washed by centrifugation over a ficoll hypaque gel cushion. The volume was then brought up to 1.0ml with buffer. Sufficient LUVET material was used to add 0.4mg of IL2/ml of methyl cellulose.

For administration, the mixture, the methyl cellulose gel containing IL2 was loaded into a 1ml syringe which was centrifuged to remove bubbles. The loaded syringe was placed at 45°C for 1 hour prior to injection.

The results of admixing interleukin with methyl cellulose are shown in TABLE I. In this table injections were intramuscular. The best results were obtained with about 6% w/v 1,5 Pa.s (1,500cp) methyl cellulose exhibiting retention/elaboration through over 10 days. In this table "K" represents hydroxypropyl methyl cellulose of 4,15 and 100 Pa.s (4,000, 15,000 and 100,000cp), "MC" represents methyl cellulose followed by the centipoise number.

Table II examines the retention and release of free interleukin in various gels. Liposomal encapsulation

5

did not reduce retention and elaboration as compared to free interleukin in similar gels. In Table II the most prolonged elaboration of interleukin was provided by methyl cellulose, particularly 1,5 Pa.s (1,500cp), and most particularly of 6% to 12%. These formulations suprisingly substantially exceded the elaboration of interleukin from ultra-high viscosity carboxyvinyl polymers (HIVIS-A is the polymer suspended in 5 mM ammonium acetate, approximately 200 Pa.s (200,000cp); HIVIS-G is the polymer suspended in glycerol approximately 1500 Pa.s (1,500,000cp)), or any other known method.

## TABLE I

### SUMMARY OF MOUSE STUDIES

#### Frequency of Positive Responses 1/

| Study | Formulation | 1h | 4h | 7h | 1d | 2d | 3d | 5d | 7d | 10d |
|---|---|---|---|---|---|---|---|---|---|---|
| 1 | Free IL2 | 6/6 | 5/5 | 5/5 | 5/5 | 2/5 | 0/5 | - | - | - |
| 2 | Free/3% MC 1500 | 6/6 | 6/6 | 6/6 | 6/6 | 6/6 | 6/6 | 1/6 | 0/6 | - |
| 3 | Free/6% MC 1500 | 6/6 | 6/6 | 5/5 | 5/5 | 5/5 | 5/5 | 3/5 | 1/5 | - |
| 4 | Free/6% MC 1500 | 6/6 | 6/6 | 6/6 | 6/6 | 6/6 | 6/6 | 6/6 | 4/4 | 2/4 |
| 5 | LUVET/6% MC 1500 | 10/10 | 10/10 | 10/10 | 10/10 | 10/10 | 9/9 | 9/9 | 6/9 | 2/9 |
| 6 | LUVET/6% MC 1500 | 6/6 | 6/6 | 6/6 | 6/6 | 6/6 | 6/6 | 6/6 | 3/6 | - |
| 7 | LUVET/1.5% MC 4000 | 6/6 | 6/6 | 6/6 | 6/6 | 5/6 | 6/6 | 3/6 | 1/6 | - |
| 8 | LUVET/3.0% MC 4000 | 6/6 | 6/6 | 6/6 | 6/6 | 6/6 | 6/6 | 6/6 | 0/6 | - |
| 9 | LUVET/2.0% K4M | 6/6 | NT | NT | 5/5 | NT | 5/5 | 2/5 | 0/5 | - |
| 10 | LUVET/2.0% K15M | 6/6 | NT | NT | 5/5 | NT | 4/5 | 2/5 | 0/5 | - |
| 11 | LUVET/2.0% K100M | 6/6 | NT | NT | 6/6 | NT | 6/6 | 3/5 | 0/6 | - |

#### Average Normalized Blood Levels 2/

| Study | Formulation | 1h | 4h | 7h | 1d | 2d | 3d | 5d | 7d | 10d |
|---|---|---|---|---|---|---|---|---|---|---|
| 1 | Free IL2 | 1027 | 109.3 | 16.9 | 1.45 | 0.39 | - | - | - | - |
| 2 | Free/3% MC 1500 | 976 | 127 | 30.7 | 1.97 | 1.8 | .67 | - | - | - |
| 3 | Free/6% MC 1500 | 720 | 104 | 53 | 3.87 | 1.73 | .67 | 0.17 | - | - |
| 4 | Free/6% MC 1500 | 1006 | 307 | 108 | 15.6 | 10 | 4.0 | 1.07 | .56 | .17 |
| 5 | LUVET/6% MC 1500 | 142 | 93 | 58 | 12 | 4.4 | 1.64 | 0.41 | .29 | .17 |
| 6 | LUVET/6% MC 1500 | 34 | 28 | 12.8 | 3.7 | 1.14 | .96 | 0.22 | .20 | - |
| 7 | LUVET/1.5% MC 4000 | 66 | 27 | 12 | 2.31 | 0.41 | .33 | 0.28 | - | - |
| 8 | LUVET/3.0% MC 4000 | 71 | 42 | 18.3 | 3.43 | 0.79 | .99 | 0.48 | - | - |
| 9 | LUVET/2.0% K4M | 163 | NT | NT | 7.03 | NT | 1.04 | 0.26 | - | - |
| 10 | LUVET/2.0% K15M | 225 | NT | NT | 5.24 | NT | 1.11 | 0.41 | - | - |
| 11 | LUVET/2.0% K100M | 167 | NT | NT | 6.02 | NT | 1.06 | 0.51 | - | - |

1/ Number of mice in each study that had a measurable IL2 blood level at a given time point.

2/ Average normalized blood levels were calculated for positive animals only (minimum n = 2) by the formula $\frac{ng/ml}{dose/animal} = 100$

## TABLE II

### SUMMARY OF FREE IL-2/GEL FORMULATIONS IN MICE

| FORMULATION | GEL | 1h | 4h | 7h | 1d | 2d | 3d | 5d | 7d | 10d |
|---|---|---|---|---|---|---|---|---|---|---|
| 1 | – | 1027 | 109 | 17 | $1.5^5$ | $.39^2$ | $-$ [2] | – | NT [3] | NT |
| 2 | 3.0% MC1500 | 976 | 31 | 31 | 2.0 | $1.8^6$ | $.7^6$ | – | – | NT |
| 3 | 6.0% MC1500 | 1006 | 307 | 108 | 16.0 | $10.0^6$ | $4.0^6$ | $1.1^6$ | $.56^6$ | $.17^2$ |
| 4 | 6.0% MC1500 | 720 | 104 | 53 | 4.0 | $1.7^5$ | $.7^5$ | $.17^3$ | – | NT |
| 5 | 9.0% MC1500 | 356 | NT | NT | 12.0 | NT | $1.8^6$ | $.24^3$ | $.17^3$ | NT |
| 6 | 12.0% MC1500 | 361 | NT | NT | 11.0 | NT | $2.4^6$ | $.46^3$ | $.22^2$ | $.28^3$ |
| 7 | 9.0% MC4000 | 372 | NT | NT | 8.0 | NT | $2.2^6$ | $.27^2$ | – | NT |
| 8 | 0.5% HIVIS-G | 504 | NT | NT | 1.5 | NT | $.14^4$ | – | – | NT |
| 9 | 1.0% HIVIS-A | 518 | NT | NT | 2.0 | NT | – | – | NT | NT |

[1] Table entries are blood levels normalized by the formula $\underline{\quad\text{ng/ml}\quad} \times 100$. Superscripts refer to the number of responding animals at that time point. In most studies six animals were tested per formulation.

[2] Below sensitivity of detection in the assay.

[3] Not tested

EP 0 390 849 B1

## Claims

1. A pharmaceutical composition of prolonged elaboration comprising methyl cellulose and an immunomodulator wherein immunomodulator is encapsulated in a liposome.

2. The composition of claim 1 wherein the immunomodulator is encapsulated in an SPLV, a FATMLV, a SUV, a LUVET, an MPV, or a MLV.

3. The composition of claim 1 or 2, wherein the immunomodulator is a colony stimulating factor, an interferon, or an interleukin.

4. A pharmaceutical composition of prolonged elaboration comprising methyl cellulose and an immunomodulator wherein the immunomodulator is a colony stimulating factor, or an interleukin, optionally IL2.

5. The composition of any one of claims 1 to 4 wherein IL2 is present in from 0.5 μg/mg to 600 μg/mg methyl cellulose.

6. The composition of any one of claims 1 to 5 wherein the methyl cellulose is from 1.0 Pa xsec (1,000 cp) to 10 Pa xsec (10,000 cp), optionally from 1.0 Pa xsec (1,000 cp) to 4 Pa xsec (4,000 cp).

7. The composition of any one of claims 1 to 6 wherein the methyl cellulose is a gel, optionally from 1% to 15% w/v and particularly from 3% to 12% w/v.

8. The composition of claim 7 wherein the methyl cellulose is 1.5 Paxsec ( 1,500 cp).

9. The composition of any one of claims 1 to 7 wherein the methyl cellulose is 6% w/v, optionally wherein the methyl cellulose is about 1.5 Pa xsec (1,500 cp).

10. The composition of any one of claims 1 to 9 comprising a parenteral, optionally subcutaneous, intramuscular or intraperitoneal vehicle.

11. A method of preparing a pharmaceutical composition of prolonged elaboration according to claims 1 to 10, comprising admixing methyl cellulose and an immunomodulator and, optionally, further comprising encapsulating said immunomodulator in a liposome when the immunomodulator is encapsulated in a liposome.

12. The method of claim 11 comprising forming a gel of methyl cellulose, optionally wherein the admixing is performed at a temperature at least below 10°C.

## Patentansprüche

1. Pharmazeutische Zusammensetzung mit verlängerter Wirkung bzw. Freisetzung (elaboration), die Methylcellulose und einen Immunmodulator enthält, wobei der Immunmodulator in ein Liposom eingekapselt ist.

2. Zusammensetzung nach Anspruch 1, worin der Immunmodulator in ein SPLV, ein FATMLV, ein SUV, ein LUVET, ein MPV oder ein MLV eingekapselt ist.

3. Zusammensetzung nach Anspruch 1 oder 2, worin der Immunmodulator ein Kolonie-stimulierender Faktor, ein Interferon oder ein Interleukin ist.

4. Pharmazeutische Zusammensetzung mit verlängerter Freisetzung, die Methylcellulose und einen Immunmodulator enthält, wobei der Immunmodulator ein Kolonie-stimulierender Faktor oder ein Interleukin, gegebenenfalls IL2, ist.

5. Zusammensetzung nach einem der Ansprüche 1 bis 4, worin IL2 in einer Menge von 0,5 bis 600 μg/mg Methylcellulose vorliegt.

6. Zusammensetzung nach einem der Ansprüche 1 bis 5, worin die Methylcellulose eine solche mit einer Viskosität von 1,0 Pa.s (1000 cP) bis 10 Pa.s (10 000 cP), gegebenenfalls von 1,0 Pa.s (1000 cP) bis 4 Pa.s (4000 cP) ist.

7. Zusammensetzung nach einem der Ansprüche 1 bis 6, worin die Methylcellulose ein Gel ist, mit einem Gehalt von gegebenenfalls 1 bis 15 Gew./Vol.-%, insbesondere 3 bis 12 Gew./Vol.-%.

8. Zusammensetzung nach Anspruch 7, worin die Methylcellulose eine Viskosität von 1,5 Pa.s (1500 cP) hat.

9. Zusammensetzung nach einem der Ansprüche 1 bis 7, welche die Methylcellulose in einer Menge von 6 Gew./Vol.-% enthält, wobei gegebenenfalls die Methylcellulose eine Viskosität von etwa 1,5 Pa.s (1500 cP) hat.

10. Zusammensetzung nach einem der Ansprüche 1 bis 9, die ein parenterales, gegebenenfalls subkutanes, intramuskuläres oder intraperitoneales Vehiculum enthält.

11. Verfahren zur Herstellung einer pharmazeutischen Zusammensetzung mit verlängerter Wirkung bzw. Freisetzung nach den Ansprüchen 1 bis 10, das umfaßt das Mischen von Methylcellulose mit einem Immunmodulator und gegebenenfalls ferner das Einkapseln des Immunmodulators in einem Liposom, wenn der Immunmodulator in einem Liposom eingekapselt ist.

12. Verfahren nach Anspruch 11, das umfaßt die Bildung eines Gels aus Methylcellulose, wobei das Mischen gegebenenfalls bei einer Temperatur von mindestens unter 10°C durchgeführt wird.

**Revendications**

1. Une composition pharmaceutique à élaboration prolongée comprenant de la méthylcellulose et un immunomodulateur, dans laquelle l'immunomodulateur est encapsulé dans un liposome.

2. La composition de la revendication 1 dans laquelle l'immunomodulateur est encapsulé dans une VPLS, une VMCED, une VUS, une LUVET, une VPM, ou une VML.

3. La composition de la revendication 1 ou 2, dans laquelle l'immunomodulateur est un facteur de stimulation de la colonisation, un interféron, ou une interleukine.

4. Une composition pharmaceutique à élaboration prolongée comprenant de la méthylcellulose et un immunomodulateur, dans laquelle l'immunomodulateur est un facteur de stimulation de la colonisation, ou une interleukine, éventullement l'IL2.

5. La composition de l'une quelconque des revendications 1 à 4 dans laquelle l'IL2 est présente en une quantité comprise entre 0,5 µg/mg et 600 µg/mg de méthylcellulose.

6. La composition de l'une quelconque des revendications 1 à 5 dans laquelle la méthylcellulose a une viscosité comprise entre 1,0 Pa xsec (1.000 cp) et 10 Pa xsec (10.000 cp), éventuellement entre 1,0 Pa xsec (1.000 cp) et 4 Pa xsec (4.000 cp).

7. La composition de l'une quelconque des revendications 1 à 6 dans laquelle la méthylcellulose forme un gel, représentant de 1 % à 15% p/v au choix et en particulier entre 3% et 12% p/v. de la composition.

8. La composition de la revendication 7 dans laquelle la méthylcellulose est à 1,5 Pa xsec (1.500 cp).

9. La composition de l'une quelconque des revendications 1 à 8 dans laquelle la méthylcellulose forme 6% p/v, éventuellement dans laquelle la méthylcellulose est à environ 1,5 Pa xsec (1.500 cp).

10. La composition de l'une quelconque des revendications 1 à 9 comprenant un vecteur parentéral, au choix sous-cutané, intramusculaire ou intrapéritonéal.

11. Un procédé de préparation d'une composition pharmaceutique à élaboration prolongée selon les revendications 1 à 10, comprenant de mélanger de la méthylcellulose et un immunomodulateur et, éventuellement, comprenant de plus d'encapsuler ledit immunomodulateur dans un liposome quand l'immunomodulateur est encapsulé dans un liposome.

12. Le procédé de la revendication 11 comprenant de former un gel de méthylcellulose, dans lequel éventuellement le mélange est pratiqué à une température au moins inférieure à 10°C.